# EUROPEAN PATENT APPLICATION

(11) **EP 2 557 153 A2**
(43) Veröffentlichungstag der Anmeldung: **13.02.2013**
(21) Anmeldenummer: 11780875.8
(22) Anmeldetag: 01.04.2011
(51) Int. Cl.: C12N 5/0735, C12N 5/079, A61P 25/00

(54) **VERFAHREN ZUR HERSTELLUNG EINER NEURALMATRIX**

(30) Priorität: 06.04.2010 RU 2010113397
(71) Anmelder: Gosudarstvennoye Obrazovatel'Noye Uchrezhdeniye Vysshyego Propyhsessional'Nogo Obrazovanija "Krasnojarskij Gosudarstvyennyj MyedItsinskij, Krasnoyarsk 660022 (RU)
(72) Erfinder: YERYEMYEYEV, Artyem Valyer'yevich, Zheleznogorsk 660084 (RU); SVYETLAKOV, Anatolij Vasil'yevich, Krasnoyarsk 660049 (RU); BOL'SHAKOV, Igor' Nikolayevich, Krasnoyarsk 660028 (RU); SHYEINA, Julija Igoryevna, Krasnoyarsk 660036 (RU); POLSTJANOJ, Alyeksyej Mikhajlovich, Krasnoyarsk 660022 (RU)
(74) Vertreter: Jeck, Anton
(86) Internationale Anmeldenummer: PCT/RU2011/000213
(87) Internationale Veröffentlichungsnummer: WO 2011/142691

(57) **Zusammenfassung**

Die Erfindung betrifft ein Verfahren zur Herstellung einer neuronalen Matrix unter Anwendung einer Kollagen-Chitosan-Unterlage und Nährböden zum Züchten von menschlichen embryonalen Stammzellen. Damit die Züchtungsqualität und -stabilität erhöht, die proliferative Aktivität der pluripotenten Stammzellen einschließlich ihrer Ausdifferenzierung in neuronale Zellen erhöht, die Immunogenität der Mikroumgebung der Zellen vermieden und die Erzeugung einer für die Transplantation geeigneten Matrix sichergestellt wird, sieht die Erfindung vor, dass die Biomasse der menschlichen embryonalen Stammzellen einer feederfreien hESKM-05-Linie zuerst in mit 0,1%iger Gelatinelösung beschichteten Flaschen angelagert wird, wobei ein koDMEM-Hauptboden verwendet und täglich gewechselt wird, dass der Hauptboden 10 % Serumersatzmittel SR, 100 µg/ml Kanamycinsulfat, 1 mM L-Glutamin-Lösung, 4 ng/ml eines Haupt-Wachstumfaktors von Fibroblasten (bFGF) und 1 mM einer essentiellen Aminosäure-Lösung enthält und dass die Biomasse danach mit Hilfe einer 0,5%igen Kollagenase-Lösung in Flaschen übertragen wird, welche eine vorbereitete Kollagen-Chitosan-Matrix in einem konditionierten Boden enthält, der von einer embryonalen neuronalen Zellkultur der Mäuse produziert wird oder aus einem Komplett-Nährboden unter Zugabe eines neuronalen N2-Faktors besteht, wobei der Boden alle drei Tage gewechselt wird.

## Beschreibung

Die Erfindung betrifft ein Verfahren zur Herstellung einer neuronalen Matrix.

Die Erfindung ist in der Biologie und Medizin und zwar in der Neurologie und Neurochirurgie, in der Zell-Nanobiotechnologie und dem Bioingenieurwesen sowie der Transplantologie einsetzbar. Sie kann zur Züchtung, Proliferation und Ausdifferenzierung der Stammzellen in neuronalen Zellen zwecks nachfolgender Transplantation angewendet werden.

Die Überlebensrate der Menschen, die eine komplizierte Verletzung der Wirbelsäule durchgemacht haben, beträgt jährlich 1500 Mann. Die Patienten bedürfen eines Operationseingriffs wegen direkter Indikationen für eine Rückenmarkrekonstruktion. Zur Zeit sind die Behandlungsverfahren solcher Patienten nur wenig effektiv, besonders wenn die Verletzung durch einen anatomischen Rückenmarkriss erschwert ist. Eine grundlegende Lösung dieses Problems kann eine Überpflanzung des autologen Nervengewebes oder die Erzeugung neuronaler Äquivalente mit hoher proliferativer Aktivität aus Stammzellen im Falle einer Nutzung von Zelltechnologien sein. Das Ziel der angemeldeten Zelltechnik (cell engineering) ist die Herstellung von solchen Bedingungen in einer Zellkultur, welche die Bildung von einem Zellenpool der neuronalen Richtung fördert. Dies kann mit Hilfe einer Unterlage bzw. einer dreidimensionalen Matrix mit Sonder-Mikroumgebung erreicht werden, welche während der Proliferation und des Ausdifferenzierens eine Nervengewebestruktur erzeugt.

Aus dem Stand der Technik sind Verfahren zur Bildung von Zellmatrizen zwecks Behandlung von spinalen Traumata unter Einsatz eines Nano-Mikrokugelsystems für die Lieferung von bioaktiven Faktoren sowie mittels der Anwendung von Mesenchymzellen aus einer Nabelader bekannt. Das erste Verfahren beruht auf der Nutzung von synthetischen Nano- und Mikrokugeln aus biologisch abbaubaren und biologisch verträglichen Kunststoffen und Copolymeren.

Die Nutzung dieser Technik basiert auf einer Induktion der Nervenregeneration im Verletzungsgebiet dank dem Chondroitinase-Enzym. Das Chondroitinase-Enzym zerstört Glykosaminglykane, die Haupthemmstoffe der Axonelongation [1- 3] sind. Darüber hinaus sind Antikörper, Neurotropine, hormonale Wachstumsfaktoren, die zur Axonverlängerung ebenfalls beitragen, in die Kunststoffnanogefüge eingebettet [4 - 6].

Durch [7] ist ein Verfahren zur Erzeugung von chirurgischen Rückenmark-Implantaten aus bioabbaubaren Kunststoffmatrizen bekannt. Solche Matrizen bilden eine Röhre, um die Enden des Rückenmarkrisses zusammenzufügen. Sie beinhalten Chondroitinase, Wachstumsfaktoren oder Schwannzellen, welche vor der Transplantation auf ein Substrat verpflanzt werden.

Dem aus [8] bekannten Verfahren liegt die Bereitung von fötalen Stammzellen zugrunde, welche auf ein Fibringel gesetzt werden, um nachher die Transplantation in den Rückenmarkrissbereich vorzunehmen. Außerdem wird das gleiche Fibringel zur Verpflanzung verwendet. Das Fibringel enthält ein fötales Rückenmark, welches mit den Auskleidungszellen des Riechnervbulbus angereichert ist. Die Anwendung dieses Verfahrens ist dadurch bedingt, dass die verpflanzten Zellen imstande sind, verschiedene Wachstumsfaktoren der Axone auszuscheiden. Außerdem fördern sie eine rasche Wiederherstellung der Bindungen zwischen den Neuronen [9].

Aus dem Stand der Technik ist auch die Nutzung einer heterogenen Kollagen-Matrix Sphärogel als Unterlage für autologe Stammzellen bekannt. Diese Matrix beinhaltet antibakterielle Präparate, Wachstums- und Ausdifferenzierensfaktoren [10, 11]. Bekanntlich werden gewebetechnische Konstruktionen für die Wiederherstellung des ZNS und des peripheren Nervensystems verwendet. So hat Bryukhovetsky mit seinen Miterfindern [12] eine gelförmige Kunststoffmatrix eingesetzt, um die Stammzellen im Verletzungsgebiet bei einem Spinaltrauma zu liefern [13]. Die dreidimensionalen Biopolymer-Konstruktionen tragen zur Erzeugung eines Transplantats bei, dessen Eigenschaften denen der biologischen Gewebe nahe sind. So wurden insbesondere Kollagenröhren oder mit Kollagengel gefüllte PGA-Röhren mit eingepflanzten Nerven- und Schwann-Zellen [14, 15] hergestellt.

Die Anwendung eines lyophilisierten Kollagengels nach dem 1. Typ sowie von Chondroitinsulfat als dreidimensionale Matrix für zelltechnische Zwecke (Lungen) wurde 2005 beschrieben [16].

Jedoch befassen sich alle o. g. Verfahren nicht mit dem Problem eines Vor-Ausdifferenzierens von auf den Matrizen gesetzten embryonalen Stammzellen in neuronale Zellen. Dagegen werden bereits fertige allogene Stammzellen oder fertige Neuronen oder die sie ersetzenden Zellen bestimmungsgemäß verwendet. Darüber hinaus verwenden die eingesetzten Matrizen keine bioverträglichen und biologisch abbaubaren sowie zell- und genschädigenden Unterlagen mit darin eingebetteten Bestandteilen eines kompletten und eines aufbereiteten neuronalen Nährbodens. Aufgrund eines solchen Ansatzes bekommen die Stammzellen keine vollwertige Mikroumgebung aus einer extrazellulären Matrix mittels immobilisierter Wachstums- und Ausdifferenzierensfaktoren mit Hilfe eines aufbereiteten Bodens. Dies schafft keine Bedingungen für ein gezieltes Ausdifferenzieren und eine Proliferation der Stammzellen. Außerdem schafft die Anwendung solcher Matrizen keine Bedingungen für eine hohe Zellhaftung (Adhäsion) an der Matrix. Das ist dadurch begleitet, dass manche Zellen beim Züchten auf der Unterlage infolge einer unzureichenden biologischen Verträglichkeit mit Bestandteilen der Unterlage verloren gehen. Die von den Erfindern vorgeschlagenen Matrizen schaffen keine Bedingungen für eine hohe Proliferation und ein Ausdifferenzieren der Stammzellen in neuronale Zellen. Darüber hinaus haben diese Matrizen kein 3D-Gefüge, um ein Funktionsgewebe aus den Abbauprodukten des Trägers zu bauen.

Eine der eventuellen negativen Folgen der Einpflanzung embryonaler Stammzellen im Körper ist die Bildung von Teratomen. Das ist auch an immundefekten (thymuslosen) Mäusen veranschaulicht worden. Die wahrscheinlichste Anwendungsform dieser Zellen in der Medizin scheint die Anwendung ihrer differenzierten Derivate zu sein. Die Druckschriften von Anissimov und der Forschungsgruppe von Pavlova zeigen, dass die Verwendung von dreidimensionalen Matrizen mit darauf gezüchteten embryonalen Stammzellen es ermöglicht, die embryonalen Stammzellen in neuronalen Zellen bei ihrer Implantation im Gehirn auszudifferenzieren, und dabei entstehen keine Teratomen. Jedoch verläuft die Transformation der embryonalen Stammzellen in die neuronalen Zellen auf den Matrizen unter Vorzüchten in einem Sonderboden mit ihrer nachfolgenden Übertragung auf die Matrix. Das erschwert die Arbeit und erhöht das Kontaminationsrisiko.

Es gibt verschiedene Verfahren zur Stimulation der embryonalen Stammzellen zwecks Bildung von Neuronen, Oligodendrozyten oder von Schwann-Mantel-Zellen.

Die Druckschriften von McDonald et al. [17] und Wichterle [18] offenbaren ein Ausdifferenzieren von embryonalen Stammzellen in Oligodendrozyte mit einer nachfolgenden Myelinisierung bei Ratten mit Spinaltrauma.

Jedoch können die vorgeschlagenen Verfahren zum Züchten von neuronalen Zellen trotz einer hohen Spezialzellenausbeute nicht wirksam für eine Transplantation angewendet werden, denn in diesen Protokollen werden Enzymlösungen (Trypsin, Kollagenase, Dispase usw.) zum Übertragen von Zellen aus Kulturflaschen benutzt. Dies bedingt eine Steigerung einer Apoptose-, einer Zelltodrate und einer Schädigungsrate eines Oberflächen-Zellreizaufnehmers [19, 20]. Die Kontaminationswahrscheinlichkeit nimmt zu, und der Transplantationsablauf ist erschwert. Das vermindert wesentlich ihre Menge und Lebensfähigkeit infolge einer Schädigung der Oberflächenreizaufnehmer während der Übertragung und Reaktionsaktivierung des vorprogrammierten Zelltods.

Die allgemeinen Nachteile der bekannten Verfahren zur Erzeugung neuronaler Zellen aus einer Stammzellenmenge sind ihr niedriger Anteil beim Züchten, ein eventuelles Malignisierungsrisiko der Stammzellen bei ihrer Transplantation ohne vorausgehende Ausdifferenzierung in Neuronen bzw. Oligodendrozyten und eine eventuelle teratogene Wirkung wegen fehlender kompletter Mikroumgebung, welche aus einer extrazellulären Matrix gebildet wird. Als seriöser Mangel ist die Gefahr einer Thrombusbildung in der Menge von gezüchteten Stamm- und neuronalen Zellen genannt. Diese Zellen haften an der Oberfläche der Matrix. Die Anwendung rekombinanter Proteine bzw. genetisch transformierter Zellen, die die Herstellungskosten solcher Matrizen erhöhen, wird von der Ausbildung einer immunologischen Antwort auf die Transplantation der Matrix nicht nur beim Einsatz einer Allozellmenge sondem auch beim Einsatz autologer Zellen begleitet. Darüber hinaus haben die bekannten Matrizen keine antibakteriellen Eigenschaften und leiten keine Gefäßneubildung ein. Die Gefäßneubildung ist notwendig, um das Nervengewebe bei den Verletzungen des ZNS und insbesondere des Rückenmarks zu bilden.

Der nächstkommmende Stand der Technik ist eine Matrix, bei der eine Kollagen-Chitosan-Konstruktion als Unterlage verwendet wird. Sie enthält aufbereitete Komplett-Nährböden. Die Unterlage weist antibakterielle Eigenschaften auf und leitet eine Gefäßneubildung ein. Sie schafft auch eine hohe biologische Verträglichkeit dank vorhandenem Chitosan mit einem hohen Azetylierungsgrad, Chondroitinsulfat, Hyaluronsäure und Heparin. Darüber hinaus erhöht die Kollagen-Chitosan-Unterlage die Züchtungsqualität und -stabilität, die proliferative Aktivität der pluripotenten Stammzellen verschiedener Herkunft, verhindert die Zyto- und Gen-Toxizität der Zellunterlage, erübrigt die Enzymbehandlung der Zellen beim Passivieren während des Nährbodenwechsels, verstärkt die Zellhaftung an der Matrixoberfläche, stellt die Herstellung einer für eine direkte Transplantation geeignete Zellmatrix sicher und verhindert eine Thrombusbildung beim Züchten der Stammzellen unter Heparin [19]. Jedoch betrifft die von den Erfindern vorgeschlagene Matrix das Verfahren zur Herstellung einer Hautzellmatrix und nicht der Zellen eines Nervensystems, da die für die Stammzellen erzeugte Mikroumgebung auf ihre Ausdifferenzierung in die Hautzellen gerichtet ist (Fibroblasten, Epidermoblasten).

Es ist Aufgabe der Erfindung, die Züchtungsqualität und -stabilität, die proliferative Aktivität der pluripotenten Stammzellen einschließlich ihrer Ausdifferenzierung in neuronale Zellen zu erhöhen, die Immunogenität der Mikroumgebung der Zellen zu vermeiden sowie die Erzeugung einer für die direkte Transplantation geeigneten neuronalen Matrix sicherzustellen.

Die gestellte Aufgabe wird durch die Merkmale des Anspruchs 1 gelöst.

Dies wird dadurch erreicht, dass die biologische Menge von menschlichen embryonalen Stammzellen einer feederfreien hESKM-05 zuerst in den Flaschen gezüchtet wird. Die Flaschen sind mit einer 0,1%igen Gelatinelösung bedeckt. Beim Züchten wird der Hauptboden koDMEM verwendet und täglich gewechselt. Der Boden enthält ein 10%iges Serumersatzmittel SR, 100 µg/ml Kanamycinsulfat, 1 mM einer L-Glutamin-Lösung, 4 ng/ml eines Haupt-Wachstumfaktors von Fibroblasten (bFGF) und 1 mM einer essentiellen Aminosäure-Lösung. Danach wird die biologische Menge mit Hilfe einer 0,5%igen Kollagenase- Lösung in die Flaschen umgesetzt. Die Flaschen enthalten eine Kollagen-Chitosan- Matrix in einem von embryonalen neuronalen Zellen von Mäusen konditionierten Boden oder einem Komplett-Nährboden unter Zugabe eines neuronalen N2-Faktors. Der Boden wird alle drei Tage gewechselt. Dabei wird eine Matrix in Form von Schwamm, Folie, Mikrokugeln, Gel oder Fasern verwendet.

Das Verfahren wird wie folgt ausgeführt. Im ersten Schritt werden menschliche pluripotente Zellen der Linie hESKM-05 verwendet (feederfreie animal-free Linie, die vom Krasnoyarsker Zentrum für reproduktive Gesundheit (ZRM) gemeinsam mit dem Institut für allgemeine Genetik der Russischen Akademie für Wissenschaften Moskau entwickelt wurde). Zuerst wird die Biomasse des Hauptbodens koDMEM unter Zugabe von 10 % SR (Serumersatzmittel), 100 µg/ml Kanamycinsulfat, 1 mM L-Glutamin, 4 ng/ml des Haupt-Wachstumfaktors der Fibroblasten (bFGF) und 1 mM der essentiellen Aminosäure-Lösung aufgewachsen. Das Aufwachsen der Zell-Biomasse wird in Flaschen vorgenommen, die mit einer 0,1%igen Gelatine-Lösung beschichtet sind. Der Bodenwechsel erfolgt täglich. Der Zustand der Kolonien wird visuell mit Hilfe eines Mikroskops Olympus BX51 ausgewertet. Im zweiten Schritt wird ein aufbereiteter Nährboden aus der embryonalen neuronalen Zellkultur hergestellt. Die Zellen werden aus 7- bis 10-tägigen Föten der rassenlosen Mäuse gewonnen. Dafür werden die Zellen bei 37° C im DMEM-Boden unter Zugabe von 10%igem embryonalen Kälberserum, 100 µg/ml Sulfat und 1 mM L-Glutamin serum, 100 µg/ml Sulfat und 1mM L-Glutamin gezüchtet. Am 3. Tag wird der Boden gesammelt und mit einem 0,22 µm Azetat-Zellulose-Filter gefiltert. Im 3. Schritt wird die Kollagen-Chitosan-Wunddecklage Kollakhit-Bol [21, 22] verwendet. Dafür wird die Wunddecklage in einem sterilen Bikarbonatpuffer voreingeweicht, um ihre Säureeigenschaften zu verringern. Nach der Neutralisation wird die Decklage dreimal mit sterilem Dulbekko-Phosphatpuffer gewaschen, in die Flaschen gesetzt und ein konditionierter (aufbereiteter) Boden zugegeben. Der aufbereitete Boden wird von der embryonalen neuronalen Zellkultur der Mäuse produziert. Alternativ wird der DMEM-Boden unter Zugabe der neuronalen Komponente N2 gemäß der Herstelleranweisung zugegeben. Oben wird auf die Kollagen-Chitosan-Wunddecklage eine Aufschwemmung von menschlichen embryonalen Stammzellen der Linie hESKM-05 aufgetragen.

Nach 5 bis 7 Züchtungstagen werden die Zellen mittels Formaldehyd fixiert, und anschließend wird eine immunozytochemische Analyse mittels Antikörper gegen saure Gliafaserprotein(GFAP)-Marker für Astrozyt, Neurofilament und Nestin durchgeführt. Die Marker werden nach einem Verfahren gemäß der Anweisung des Antikörper-Herstellers erkannt. Die Zellkerne werden mit DAPI (0,1 µg/ml) im Laufe von 10 Minuten gefärbt. Um die Bilder zu bekommen und die Analyse durchzuführen, werden ein Fluoreszenzmikroskop Olympus BX-51 sowie Softwareprodukte von Applied Spectral Imaging (USA) angewendet. Zwecks Analyse jedes Markers wird der Versuch dreimal wiederholt, wobei die neuronalen Zellen in drei Flaschen aufgewachsen sind. In jeder der Flaschen werden stichprobenweise 6 Zonen ausgesondert, um eine Analyse einer immunozytochemischen Reaktion durchzuführen. Die Mikroskopierung jeder Zone wird in 30 Blickfeldern durchgeführt.

Die Ergebnisse der morphologischen Analyse der Matrix haben gezeigt, dass der neuronale Zellengehalt auf einer 30 cm² großen Unterlage ca. 7 Mio. beträgt.

Die Erfindung wird anhand der Zeichnungen näher erläutert. Es zeigen:

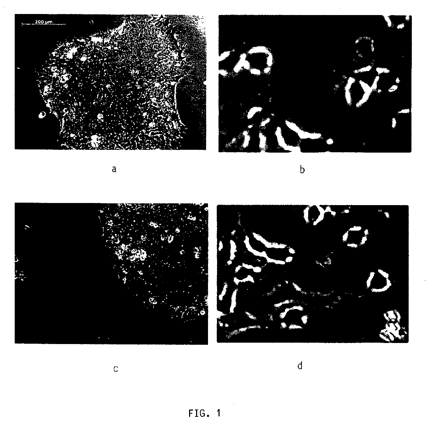

- Fig. 1: die Ergebnisse der Phasenkontrastmikroskopie der gezüchteten menschlichen embryonalen Stammzellen, die auf den Kollagen-Chitosan-Matrizen im Laufe von 1 bis 14 Tagen unter dem aufbereiteten Boden (a - am 1. Tag, b - am 14. Tag) passiviert werden. Die embryonalen Stammzellen werden im neuronalen Bereich beim Einsatz des neuronalen aufbereiteten Bodens oder des DMEM-Bodens unter Zugabe der neuronalen Komponente N2 am 1. und am 14. Tag beim Züchten auf der Kollagen-Chitosan-Matrix ausdifferenziert: Es entstehen lebensfähige spindelförmige Zellen mit Eigenzellausläufern, welche Interzellulärbindungen bilden (c - am 1. Tag, d - am 14. Tag).

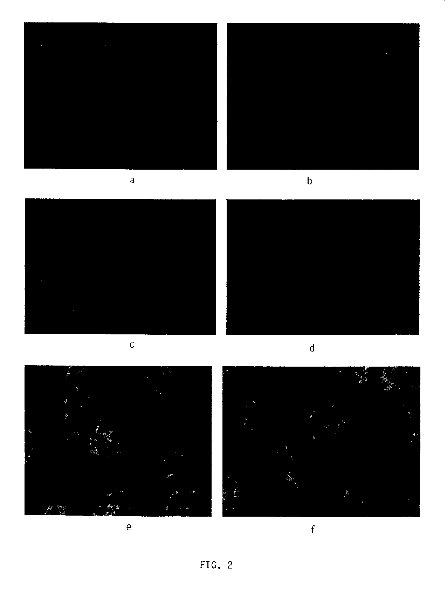

- Fig. 2: den Expressionsverlauf von Neurofilament am 1., 7. und 14. Tag in den am Kollagen-Chitosan-Komplex unter vorhandenem aufbereiteten Boden (a, b, c) oder unter Zugabe von N2-Komplex (d, e, f) gezüchteten Zellen. Die morphologische Forschung hat gezeigt, dass die embryonalen Stammzellen nach der Züchtung unter obigen Bedingungen imstande sind, sich den neuronalen Phänotyp anzueignen. Die immunozytochemische Analyse der embryonalen Stammzellen während der Ausdifferenzierung weist die Expression von einem der neuronalen Marker, und zwar von Neurofilament, nach. Es ist festzustellen, dass die Fluoreszenzsignale des genannten Markers am 1. Tag in beiden Fällen fehlen. Das Neurofilament ist am 5. bis 7. Tag der Züchtung sowohl im konditionierten Boden als auch im durch N2-Faktor ergänzten Boden (Fig. 2) zu erkennen.

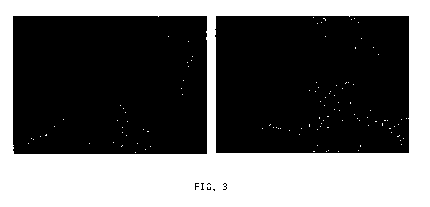

- Fig. 3: die immunozytochemische Analyse der embryonalen Stammzellen im konditionierten Boden. Der konditionierte Boden wurde infolge der Züchtung der embryonalen neuronalen Zellen der Mäuse (a, b, c) oder infolge der Zugabe der N2-Komponente (d, e, f) aufbereitet. Die Zellen wurden mittels eines 1%igen Formaldehyds im Phosphatpuffer fixiert und mittels Antikörper gegen das saure Gliafaserprotein (a, d), Nestin (b, e) und Neurofilament (c, f) behandelt. Danach wurden sie mittels sekundärer Antikörper markiert, und die Fluoreszenz wurde erkannt. Die GFAP-, Nestin- und Neurofilament-Expression ließ sich bereits am 5. Tag beim Züchten der embryonalen Stammzellen in dem durch embryonale neuronale Zellen konditionierten Boden erkennen. Am 7. Tag wurde sie auch in den Zellen festgestellt, die in dem durch N2-Komponente ergänzten Boden gezüchtet wurden.

Somit erhöht das vorgeschlagene Verfahren zur Herstellung einer neuronalen Matrix die Züchtungsqualität und -stabilität sowie die proliferative Aktivität der pluripotenten Stammzellen einschließlich ihrer Ausdifferenzierung in die neuronalen Zellen. Es verhindert die Immunogenität der Mikroumgebung der Zellen und stellt die Herstellung einer Zellmatrix sicher, die für eine direkte Transplantation geeignet ist.

### Informationsquellen

1. Zuo J. Degradation of chondroitin sulfate proteoglycan enhances the neuritepromoting potential of spinal cord tissue / J. Zuo, D. Neubauer, K. Dyess, T.A. Ferguson, D. Muir // Exp. Neurol - 1998. - -Vol.154. - P. 654-662.
2. Moon L.D., Regeneration of CNS axons back to their target following treatment of adult rat brain with chondroitinase ABC / L.D. Moon, R.A. Asher, K.E. Rhodes, J.W. Fawcett // Nat. Neurosci. - 2001. - Vol. 4. - P. 465-466.
3. Bradbury E.J. Chondroitinase ABC promotes functional recovery after spinal cord injury / E.J. Bradbury, L.D. Moon, R.J. Popat, V.R. King, G.S. Bennett, P.N. Patel, J.W. Fawcett, S.B. McMahon // Nature - 2002. - Vol. 416. - P. 636-640.
4. Bregman B.S. NeurotiOphic factors increase axonal growth after spinal cord injury and transplantation in the adult rat / B.S. Bregman, M. McAtee, H.N. Dai, P.L. Kuhn / Exp. Neurol. - 1997. - Vol. 148. - P. 475-494.
5. Kobayashi BDNF and NT-4/5 prevent atrophy of rat rubrospinal neurons after cervical axotomy, stimulate GAP-43 and Tal- tubuln mRNA expression and promote axonal regeneration / Kobayashi et. al. // J Neurosci. - 1997. - Vol. 17. - P. 9583-9595.
6. Liu Y. Transplants of fibroblasts genetically modified to express BDNF promote regeneration of adult rubrospinal axons and recovery of forelimb recovery / Y. Liu, D. Kim, B.T. Himes, S.Y. Chow, T. Schallert, M. Murray, A. Tessler, I. Fischer//J. Neurosci. - 1999. - Vol.19. - P. 4370-4387.
7. US Patent 7163545, 16.01.2007 www.freepatentsonline.
8. Patent RU 2195941. IPC⁷ A61 K35/48, A61 K35/54, A61 B17/00, BIPM (Informationsblatt von Rospatent "Erfindungen. Gebrauchsmuster") No. 1, 10.01.2003.
9. S.S. Rabinovich. Transplantation von Stammzellen bei der Behandlung von Rückenmarkverletzungen / S.S. Rabinovich, V.I. Seledtsov, O.V. Povlyutchenko, V.V. Sen'yukov, S.A. Savchenko, I.A. Volchek, V.I. Yarokhno, N.G. Kolossov, V.A. // Informationsblatt der Akademie für Militärmedizin (Vestnik voyennomeditsinskoy akademii). - 2003. - B.2. - S.:121-126.
10. Patent RU 2249462 IPC⁷ A61 K38/39, A61 K35/12, BIPM Nr. 10, 10.04.2005.
11. Patent RU 2316334 IPC⁸ A61 K35/64, A61 K36/18, A61 B5/05, BIPM Nr. 4, 10.02.2008.
12. I.S. Bryukhovetsky, S.O. Ushakov. Klinisch- pathogenetische Begründung der Anwendung von menschlichen Fötalgewebe bei ZNS Erkrankungen. // S. Aufsatzsammlung "Transplantation von menschlichen Fötalgewebe und -Zellen". M.-1996.- S. 53-56.
13. I.S. Bryukhovetsky, I.V. Diuzen, P.A. Motavin. Morphochemische Charakteristik des Rückenmarkes von Ratten nach Thorax- Lungensegmentektomie und Transplantation der Kollagen-Kunststoffmatrix Sphärogel-E mit eingegliederten neuroepithelialen Belegzellen. // Zellentransplantation und Gewebetechnik. - 2008.- B.3.- Heft 22.- S.57-62.
14. Bryan D.J., Tang J.B., Doherty S.A., Hile D.D., Trantolo D.J., Wise D.L., Summerhayes I.C. Enhanced peripheral nerve regeneration through a poled bioresorbable poly(lactic-co-glycolic acid) guidance channel.// J. Neural. Eng.-2004.-Vol.I(2).-P.91-98.
15. Rutkowski G.E., Miller C.A., Jeftinija S., Mallapragada S.K. Synergistic effects of micropatterned biodegradable conduits and Schwann cells on sciatic nerve regeneration. //J. Neural. Eng.-2004.-Vol.I.-P.151-157.
16. Chen P., Marsilio E., Goldstein R.H., Yannas I.V., Spector M. Formation of lung alveolar-like structures in collagen-glycosaminoglycan scaffolds in vitro. //J.Tissue Eng.-2005.-Vol.II(9-10).- P.1436-1448.
17. McDonald J.W., Liu X.Z., Qu Y. Transplanted embryonic stem cells survive, diffentiate, and promote recovery in injured rat spinal cord. // Nat.Med.-1999.-5(12).-P.1410-1412.
18. Wichterle H, Lieberam I, Porter JA, Jessell TM. Directed differentiation of embryonic stem cells into motor neurons. //Cell.- 2002.-Vol.II0(3).-P.385-397.
19. Yeremeev A.V., Zotova N.V., Vlasov A.A., Bolschakov I.N., Svetlakov A.V. Verfahren zur Herstellung von Haut-Zellmatrix. Patentanmeldung 2008131742/15 IPC⁸ C12 N5/00, BIPM Nr. 4, 10.02.2010.
20. Yeremeev A.V., Svetlakov A.V., Bolschakov I.N., Vlasov A.A., Arapova V.A. Lebensfähigkeit und Funktionen der pluripotenten Zellen und Fibroblasten von der Dermal- und Epidermalschicht der Tiere unter Züchtungsbedingungen an den Kollagen-Chitosan-Decklagen // Sibirskoye Medizinskoye Obozrenie. -2008, Heft 6(54).- S.24-27.
21. Patent RU 2 252 787, IPC⁷ A61 L15/28, A61 L 15/32, A61 L27/60, BIPM No. 15, 27.05.2005.
22. Patent RU 2 254 145, IPC⁷ A61 L15/28, A61 L 15/32, A61 L 26/00, BIPM No. 17, 20.06.2005.

## Patentansprüche

1. Verfahren zur Herstellung einer neuronalen Matrix unter Anwendung einer Kollagen-Chitosan-Unterlage und Nährböden zum Züchten von menschlichen embryonalen Stammzellen,
**dadurch gekennzeichnet,**
**dass** die Biomasse der menschlichen embryonalen Stammzellen einer feederfreien hESKM-05-Linie zuerst in mit 0,1%iger Gelatinelösung beschichteten Flaschen angelagert wird, wobei ein koDMEM-Hauptboden verwendet und täglich gewechselt wird,
**dass** der Hauptboden 10 % Serumersatzmittel SR, 100 µg/ml Kanamycinsulfat, 1mM L-Glutamin-Lösung, 4 ng/ml eines Haupt-Wachstumfaktors von Fibroblasten (bFGF) und 1 mM einer essentiellen Aminosäure-Lösung enthält und
**dass** die Biomasse danach mit Hilfe einer 0,5%igen Kollagenase-Lösung in Flaschen übertragen wird, welche eine vorbereitete Kollagen-Chitosan-Matrix in einem konditionierten Boden enthält, der von einer embryonalen neuronalen Zellkultur der Mäuse produziert wird oder aus einem Komplett-Nährboden unter Zugabe eines neuronalen N2-Faktors besteht, wobei der Boden alle drei Tage gewechselt wird.

2. Verfahren nach Anspruch 1,
**dadurch gekennzeichnet,**
**dass** die Kollagen-Chitosan-Matrix in Form eines Schwamms, einer Folie, von Mikrokugeln, Gel oder Fasern verwendet wird.
